Europäisches Patentamt

(19) European Patent Office

Office européen des brevets

(11) Numéro de publication: **0 029 790**
**B1**

(12) **FASCICULE DE BREVET EUROPEEN**

(45) Date de publication du fascicule du brevet: **27.03.85**

(51) Int. Cl.⁴: **A 61 K 31/615**

(21) Numéro de dépôt: **80401679.8**

(22) Date de dépôt: **21.11.80**

(54) **Nouveau médicament à base d'aspirine et d'heptaminol.**

(30) Priorité: **26.11.79 FR 7929036**

(43) Date de publication de la demande:
**03.06.81 Bulletin 81/22**

(45) Mention de la délivrance du brevet:
**27.03.85 Bulletin 85/13**

(84) Etats contractants désignés:
**AT BE CH DE FR GB IT LI LU NL SE**

(56) Documents cités:
**DE-A-2 504 546**
**FR-M- 4 822**
**GB-A- 915 813**

**THE MERCK INDEX, edition 9, 1976 Merck &
Co., ref.: 4518, page 609 RAHWAY, N.J. (US)
"Heptaminol, Hydrochloride"**

(73) Titulaire: **SOCIETE D'ETUDES SCIENTIFIQUES
ET INDUSTRIELLES DE L'ILE DE FRANCE
46, Boulevard de Latour-Maubourg
F-75340 Paris Cedex 07 (FR)**

(72) Inventeur: **Perrot, Jacques
13, boulevard Henri IV
F-75004 Paris (FR)**

(74) Mandataire: **Gallochat, Alain
SOCIETE D'ETUDES SCIENTIFIQUES ET
INDUSTRIELLES DE L'ILE DE FRANCE 46,
Boulevard de Latour Maubourg
F-75340 Paris Cedex 07 (FR)**

Courier Press, Leamington Spa, England.

# 0 029 790

**Description**

La présente invention concerne un nouveau médicament à base d'Aspirine et d'Heptaminol (c'est-à-dire du 6-amino-2-méthyl-2-heptanol).

L'Aspirine, ou acide acétylsalicylique, est utilisée habituellement comme analgésique; l'Heptaminol, plus spécialement sous sa forme de chlorhydrate, est essentiellement appliqué comme cardiotonique, les effets stimulants de l'Heptaminol sur le système cardiovasculaire permettant à ce composé de servir comme correcteur des déficiences cardiaques et de certaines hypotensions. Les effets de l'Aspirine et de l'Heptaminol sont donc disjoints.

On connaît, d'après le brevet DE 2 504 546, une composition pharmaceutique contenant de l'Aspirine et un sel d'Heptaminol, le p-chlorophénoxyacétate d'Heptaminol.

On connaît également, d'après le brevet GB 915 813, des compositions pharmaceutiques à base d'alcanolamines dont les propriétés antianaphylactiques sont renforcées par différents types d'agents thérapeutiques, tels que des agents antiinflammatoires, en particulier l'Aspirine.

Cependant, alors que dans les premier cas, il s'agit d'un simple mélange de composés dont les propriétés ne sont ni modifiées ni renforcées par rapport à celles des constituants utilisés séparément et que, dans le second cas, il s'agit d'une association où les propriétés de l'un des deux constituants seulement, celles de l'alcanolamine, sont renforcées, il s'est révélé que l'association Aspirine-Heptaminol aboutissait à des résultats surprenants et en tout cas supérieurs à ceux obtenus par l'Aspirine seule ou par l'Heptaminol seul.

C'est ainsi que l'effet analgésique chez la souris a été étudié avec l'association Aspirine-Heptaminol d'une part et avec l'Aspirine seule d'autre part. Cette recherche a été effectuée par voie orale en utilisant le test à la phénylbenzoquinone.

L'activité analgésique de l'association Aspirine-Heptaminol dans un rapport 2/3-1/3 a été jugée par la diminution, chez la souris traitée, du nombre de "tortillements" que provoque l'injection intrapéritonéale de 2-phényl 1,4 benzoquinone. Chaque groupe expérimental comportait 15 animaux répartis au hasard, de souche NMRI-Han, âgés de 5 semaines environ et pesant entre 20 et 22 grammes. L'ensemble de cette étude a porté sur 75 souris mâles dont 10 ont servi de "contrôles".

Ce test a été utilisé selon les modalités expérimentales décrites par H. Blumberg et Coll. (Proc. Soc. Exp. Biol. and Med. 1965, 118, *3*: 763—766), c'est-à-dire que chaque manifestation motrice est comptée pour un point et que le nombre total de "tortillements" de chaque animal est noté durant les 20 minutes qui suivent l'injection de phénylbenzoquinone. La valeur "contrôle" est déterminée à partir du nombre moyen noté chez les animaux sans traitement étudiés le même jour; le calcul de l'activité analgésique est basé sur le pourcentage d'animaux qui a montré seulement moins de 50 p. 100 du nombre moyen de torsions du groupe sans traitement.

La dose analgésique 50 (DA 50) et ses limites de confiance pour $P=0,05$ sont alors déterminées selon la méthode graphique de J. T. Litchfield et F. Wilcoxon (J. Pharm. Exp. Therm. 1949, *96*: 99—113). Les résultats figurent dans les tableaux ci-dessous:

| Doses mg/kg (voie orale) | Nombre d'animaux | Nombre d'animaux protégés | % D'animaux protégés |
|---|---|---|---|
| | | à l'égard de la phénylbenzoquinone | |
| 18,57 | 15 | 3 | 20,0 |
| 37,5 | 15 | 6 | 40,0 |
| 75,0 | 15 | 8 | 53,3 |
| 150,0 | 15 | 12 | 80,0 |

> Dose analgésique 50 (DA 50): 54,00 mg/kg
> Limite inférieure: 34,74 mg/kg
> Limite supérieure: 83,93 mg/kg

La DA 50 de l'association exprimée en Aspirine est alors de 36 mg/kg avec des limites de confiance égales à 23,16 et 55,95 mg/kg.

Il convient de rappeler que dans les mêmes conditions expérimentales, la dose analgésique 50 (DA 50) de l'acide acétylsalicylique est égale à 145 mg/kg avec des limites de confiance égales à 91,1 et 230,8 mg/kg pour $P=0,05$.

La comparaison des doses analgésiques 50 de l'Aspirine avec l'association Aspirine-Heptaminol donne un rapport égal à:

2

## 0 029 790

$$2,69 \ \frac{\text{DA 50 Aspirine}=145}{\text{DA 50 Aspirine-Heptaminol}=54}$$

les deux droites étant parallèles, avec des limites de confiance pour P=0,05 égales a 1,417 et 5,105. Si on exprime maintenant la DA 50 de l'association Aspirine-Heptaminol en Aspirine le rapport 145/36 est égal alors à 4,03 avec des limites de confiance pour P=0,05 égales à 2,123 et 7,649.

Il est donc possible de dire que, dans ces conditions expérimentales, l'association Aspirine-Heptaminol est 2,69 fois plus active que l'Aspirine seule et qu'en outre la quantité nécessaire d'Aspirine à l'obtention d'un même effet analgésique est quatre fois moins élevée lorsque les animaux recoivent l'association Aspirine-Heptaminol.

D'autre part il a également été compté le nombre total de manifestations motrices douloureuses ("tortillements") de chaque animal et déterminé pour chaque série la valeur moyenne (avec erreur-type de la moyenne). On a alors comparé les moyenns observées dans les séries traitées à celle notée dans le groupe "contrôle" en utilisant le test "t" de Fisher-Student pour séries homogènes.

| | Doses mg/kg en association | | | | |
|---|---|---|---|---|---|
| | 0 | 18,75 | 37,5 | 75,0 | 150,0 |
| Moyenne du nombre de tortillements | 94,9±9,66 | 74,9±9,37 | 51,2±6,57 | 41,7±8,13 | 32,1±6,93 |
| Pourcentage de diminution du nombre de tortillements | | −21,1 | −46,0 | −56,1 | −66,2 |
| Degré de signification ẍ | | non significatif | 0,001 | 0,001 | 0,001 |
| Pourcentage de souris "protégées" selon Blumberg | 6,7 | 20,0 | 40,0 | 53,3 | 80,0 |
| ẍ: Selon le test "t" de Fisher-Student pour séries homogènes (2 bornes). | | | | | |

Les résultats figurant dans le tableau ci-dessus montrent que le nombre moyen de "tortillements" induits par l'injection intrapéritonéale de phénylbenzoquinone est diminué de 46 p. 100 dès la dose de 37,5 mg/kg de l'association (P=0,001), d'une manière proportionnelle à la posologie pour atteindre une différence de 66,2 p. 100 à 150 mg/kg de l'association, par rapport à la série "contrôle". Le pourcentage d'animaux "protégés" selon Blumberg augmente proportionnellement à la posologie pour atteindre 80 p. 100 à la dose de 150 mg/kg de l'association.

Des essais cliniques ont également été menés selon la méthode du double insu croisé, entre l'Aspirine seule et l'association Aspirine-Heptaminol.

Lors de ces essais, l'association se présentait sous forme de comprimés dosés à 350 mg d'acide acétylsalicylique microencapsulé et à 187 mg de chlorhydrate d'Heptaminol correspondant à 150 mg d'Heptaminol base, répondant à la formule:

Principe actif:
ASPIRINE MICROENCAPSULEE
Exprimé en produit pur     300 mg
CHLORHYDRATE D'HEPTAMINOL
Exprimé en base     150 mg
Excipients:
Méthylcellulose 1500 cps,
Lévilite, Cellulose microcristalline     680 mg
(Avicel PH 102), Saccharinate de sodium,
Acide stéarique pulvérisé, q.s.p.

L'Aspirine se présentait sous forme de comprimés dosés à 300 mg d'acide acétylsalicylique microencapsulé d'aspect identique à celui de l'association.

3

Principe actif:

ASPIRINE MICROENCAPSULEE

Exprimé en produit pur                                                     300 mg

Excipients:

Mannitol, Cellulose microcristalline                                        680 mg

(Avicel PH 102), Méthylcellulose 1500 cps,

Silice (lévilite), Saccharinate de sodium,

Acide stéarique, q.s.p.

Ces essais ont été menés chez des malades souffrant d'algies rhumatismales chroniques, et ont consisté en une comparaison de l'effet thérapeutique, de la tolérance clinique et biologique de l'association Aspirine-Heptaminol avec l'Aspirine seule.

Au terme de ces essais, l'analyse statistique des résultats cliniques a permis d'arriver aux conclusions suivantes:

1. Les deux produits, Aspirine seule et association, présentent une activité statistiquement significative sur la douleur, avec une supériorité d'action de l'association ($p < 0,02$).

2. Il n'existe aucune différence significative entre la tolérance clinique et biologique de l'association et de l'Aspirine seule.

Cliniquement il est donc possible d'affirmer que l'association améliore nettement les qualités de l'Aspirine sans en majorer les inconvénients.

Il ressort de tout ce qui précède que l'effet de l'Aspirine est largement augmenté dans l'association Aspirine-Heptaminol.

Il a été également montré que l'effet de l'Heptaminol était augmenté dans l'association Aspirine-Heptaminol, au niveau de l'antiagrégation plaquettaire, notamment lors des embolies veineuses.

A cet effet des expériences ont été menées "in vivo" chez le lapin anesthésié chez lequel une ouverture de la boîte crânienne est réalisée au niveau de l'os pariétal. La dure-mère ainsi mise à nu et délicatement soulevée est incisée en croix. Pendant toute la durée de l'expérience, le champ opératoire est recouvert d'un film de sérum physiologique (solution aqueuse de NaCl à 9 p. 1000) porté à 37°C. La tête de l'animal est maintenue en position horizontale dans un appareil de contention et l'observation de la circulation piale est réalisée à l'aide d'une loupe binoculaire de Wild avec un grossissement×60.

La formation de thrombi veineux est provoquée par application directe de lactate de sodium sur la surface du cortex; le temps de contact est fixé à 1 minute 30; puis le lactate est retiré par aspiration et le champ rincé au sérum physiologique.

Les veines piales qu'on peut observer après ouverture de la dure-mère ont été réparties en fonction de leur diamètre afin de pouvoir les classer plus aisément en plusieurs catégories: veines d'ordre I (celles dont le diamètre est le plus important), veines d'ordre II, veines d'ordre III et veinules. Les veines d'ordre I, au nombre de 1 ou 2 seulement par champ, n'ont pas été prises en considération; les veinules, trop fines pour permettre une observation discriminative au grossissement×60, ont également été éliminées des critères de jugement. Selon les animaux, les veines d'ordre II sont représentées au nombre de 4 à 10 par champ observé; les veines III au nombre de 10 à 30 selon les cas.

Après application de lactate de sodium, on note le délai d'apparition du premier thrombus dans les veines d'ordre II et d'ordre III.

Puis respectivement 15 et 30 minutes après cette application, on relève l'état d'embolisation de la vascularisation veineuse piale avec pour chaque catégorie de vaisseaux une subdivision en veines "intactes" et en veines "pathologiques" dans lesquelles apparaissent des emboles de thrombi plaquettaires.

De façon à avoir des résultats comparables, trois séries d'expérience ont été menées:

a) Série contrôle

L'application topique de lactate de sodium sur la surface du cortex dans 10 champs vasculaires permet d'observer 15 minutes plus tard l'embolisation de 35,4 p. 100 des veines d'ordre II, de fort calibre, et celle de 45,3 p. 100 d'entre-elles à la 30ème minute. A l'égard des veines de diamètre plus fin (veines III) on enregistre la présence de thrombi plaquettaires dans 67,8 p. 100 des cas 15 minutes après application de lactate et dans 70,7 p. 100 des cas à la 30ème minute.

b) Séries "Heptaminol"

L'administration orale préventive d'Heptaminol durant 3 jours consécutifs ne modifie pas l'importance de l'embolisation des veines d'ordre II de fort calibre. On observe même à forte dose (50 et encore 12,5 mg/kg) une recrudescence du nombre de veines dans lesquelles on dénombre des agrégats plaquettaires après application du lactate.

A l'égard des veines de diamètre plus fin (veines d'ordre III), l'Heptaminol exerce un effet protecteur; la diminution la plus marquée du pourcentage de veines III embolisées s'observe dans les séries traitées par 6,25 mg/kg et 3,125 mg/kg d'Heptaminol.

# 0 029 790

c) Séries Aspirine+Heptaminol

L'association d'Aspirine à l'Heptaminol accroît le pouvoir anti-agrégant plaquettaire de ce dernier. Alors que l'Heptaminol seul est dénué d'activité à l'égard des veines d'ordre II, l'association Aspirine+Heptaminol, quelle que soit la dose, réduit de façon nette l'embolisation des veine piales; la diminution maximum et statistiquement significative du nombre de veines "atteintes" est obtenue dans la série "Aspirine 12,5 mg/kg+Heptaminol 6,25 mg/kg" (P=0,005 à la 30ème minute).

A l'égard des veines d'ordre III, plus fines, on retrouve un phénomène analogue. L'adjonction d'Aspirine augmente l'intensité du pouvoir protecteur de l'Heptaminol envers l'apparition d'agrégats plaquettaires dans les veines piales de petit diamètre; l'effet thérapeutique maximum semble là encore atteint en présence de l'association: Aspirine (12,5 mg/kg)+Heptaminol (6,25 mg/kg).

Il ressort de ce qui précède que l'effet antiagrégant plaquettaire de l'Heptaminol est augmenté dans l'association Aspirine-Heptaminol.

D'autres essais ont été conduits mettant en évidence les propriétés de ladite association.

C'est ainsi que cette dernière ne modifie pas l'électrogénèse corticale du lapin vigile; on ne peut donc pas attribuer à cette association des effets stimulants centraux évoquant une action amphétaminique dont l'intérêt thérapeutique pourrait être contesté.

En outre, l'expertise toxicologique de l'association Aspirine-Heptaminol ne révèle que les effets irritants connus de l'Aspirine à hautes doses sur la muqueuse du tractus digestif. Cette expertise a été menée par voie orale, chez le chien et chez le rat pendant 3 mois; l'adjonction d'Heptaminol à l'Aspirine n'augmente pas les effets irritants connus de cette dernière.

Au niveau de la toxicité aigüe, la DL 50 a été déterminée chez la souris et chez le rat selon la méthode PROBIT; les résultats sont les suivants:

— chez la souris

|  | DL 50 (g/kg) |
|---|---|
| Aspirine | 1,49 |
| Chlorhydrate d'Heptaminol | 3,97 |
| association* | 2,39 |

* 2,39 g d'association correspondent à 1 054 mg d'Aspirine et 527 mg de chlorhydrate d'Heptaminol.

La toxicité de l'association résulte simplement de l'addition sans potentialisation de la toxicité des deux composants.

— chez le rat

|  | DL 50 (g/kg) |
|---|---|
| Aspirine | 1,87 |
| Chlorhydrate d'Heptaminol | 9,08 |
| association* | 6,99 |

* 6,99 g d'association correspondent à 3,083 g d'Aspirine et 1,541 g de chlorhydrate d'Heptaminol.

La toxicité de l'association est légèrement inférieure à la somme des toxicités (inhibition) des deux composants.

En ce qui concerne la proportion d'Aspirine et d'Heptaminol dans l'association, le rapport 2/3—1/3 précité a été préféré.

Par ailleurs, il peut se révéler intéressant d'isoler l'Aspirine de l'Heptaminol de façon à éviter une désacétylation éventuelle de l'Aspirine; à cette fin, l'excipient comportera une substance permettant "l'isolation" des deux constituants principaux. La microencapsulation de l'Aspirine est l'un des moyens réalisant cette isolation.

## Revendications pour les Etats Contractants: BE, CH, DE, FR, GB, IT, LU, LI, NL, SE

1. Médicament nouveau caractérisé en ce qu'il comprend en association, 2/3 en poids l'Aspirine et 1/3 en poids d'heptaminol, sous forme se base ou de chlorhydrate.

2. Composition pharmaceutique caractérisée en ce qu'elle comprend comme principe actif le médicament selon la revendication 1, associé à un support pharmaceutiquement acceptable.

'5

**0 029 790**

3. Composition pharmaceutique selon la revendication 2 caractérisée en ce qu'elle se présente sous forme de comprimés comprenant 300 mg d'Aspirine pure, 150 mg de chlorhydrate d'Heptaminol exprimé en base, des excipients en quantité suffisante pour 680 mg.

**Revendications pour l'Etat Contractant: AT**

1. Procédé de préparation d'une composition pharmaceutique qui consiste à mélanger deux parties en poids d'Aspirine, à une partie d'heptaminol pris sous forme de base ou sous forme de son équivalent en chlorhydrate, puis à mélanger le produit ainsi obtenu à un support pharmaceutiquement acceptable.

2. Procédé selon la revendication 1 , qui consiste à microencapsuler l'Aspirine avant de procéder au mélange avec l'heptaminol base ou le chlorhydrate d'heptaminol.

3. Procédé selon les revendications 1 ou 2, qui consiste à comprimer le mélange obtenu, en unités contenant 300 mg d'Aspirine, 150 mg de chlorhydrate d'heptaminol et 680 mg d'excipient.

**Patentansprüche fur die Vertragsstaaten: BE, CH, DE, FR, GB, IT, LI, LU, NL, SE**

1. Neues Arzneimittel, enthaltend eine Kombination von 2/3 Gew.-Teilen Aspirin und 1/3 Gew.-Teilen Heptaminol.

2. Pharmazeutische Zusammensetzung, dadurch gekennzeichnet, daß sie als Wirkstoff das Arzneimittel nach Anspruch 1 zusammen mit einem pharmazeutisch verträglichen Träger enthält.

3. Pharmazeutische Zusammensetzung nach Anspruch 2, dadurch gekennzeichnet, daß sie in Form von Tabletten vorliegt, die 300 mg Aspirin (Reinsubstanz), die äquivalente Menge Heptaminol Chlorhydrat von 150 mg seiner Base sowie die ausreichende Menge an Excipienten um zu 680 mg erreichen, enthalten.

**Patentansprüche für den Vertragsstaat: AT**

1. Verfahren zur Herstellung einer pharmazeutischen Zusammensetzung, dadurch gekennzeichnet, daß man 2 Gew.-Teile Aspirin mit 1 Gew.-Teil Heptaminol in Form der Base desselben oder in Form einer äquivalenten Menge seines Chlorhydrates mischt und anschließend das so erhaltene Produkt mit einem pharmazeutisch verträglichen Träger vermischt.

2. Verfahren nach Anspruch 1, dadurch gekennzeichnet, daß man Aspirin vor dessen Vermischen mit der Heptaminolbase oder dem Chlorhydrat derselben mikroverkapselt.

3. Verfahren nach Anspruch 1 oder 2, dadurch gekennzeichnet, daß man das so erhaltene Gemisch zu Einheiten, die 300 mg Aspirin, 150 mg Heptaminol base in Form der äquivalent Menge seines Chlorhydrates und genug Exzipient um zu 680 mg erreichen enthalten, komprimiert.

**Claims for the Contracting States: BE, CH, DE, FR, GB, IT, LI, LU, NL, SE**

1. A novel medicament characterised in that it comprises, in association, 2/3 by weight of Aspirin and 1/3 by weight of heptaminol, in base or hydrochloride form.

2. A pharmaceutical composition characterised in that it comprises as active ingredient the medicament according to claim 1 associated with a pharmaceutically acceptable carrier.

3. A pharmaceutical composition according to claim 2 characterised in that it is in the form of compressed tablets comprising 300 mg of pure Aspirin, 150 mg of Heptaminol hydrochloride, expressed in base terms, and excipients q.s. for 680 mg.

**Claims for the Contracting State: AT**

1. Process for preparing a pharmaceutical composition which consists in mixing 2 parts in weight of Aspirin with 1 part in weight of Heptaminol under the form of base or of the equivalent amount of its hydrochloride and then mixing the resulting product with a pharmaceutically acceptable excipient.

2. Process according to claim 1 which consists in microencapsulating Aspirin before its mixing with Heptaminol base or hydrochloride.

3. Process according to claims 1 or 2 which consists in compressing the resulting mixture into unit forms containing 300 mg of Aspirin, 150 mg of Heptaminol (under the form of hydrochloride) and excipients in sufficient amount for 680 mg.